# EUROPÄISCHE PATENTANMELDUNG

(11) **EP 2 316 547 A1**
(43) Veröffentlichungstag der Anmeldung: **04.05.2011**
(21) Anmeldenummer: 10008408.6
(22) Anmeldetag: 12.08.2010
(51) Int. Cl.: A63B 71/06, A63B 24/00

(54) **Verfahren und Anordnung zum interaktiven Fitnesstraining**

(30) Priorität: 30.10.2009 DE 102009051437
(71) Anmelder: Deutsche Telekom AG, 53113 Bonn (DE); Trunz-Carlisi, Elmar, 50999 Köln (DE); Ochs, Sebastian, 50999 Köln (DE)
(72) Erfinder: Delloch, Manon, 13439 Berlin (DE); Trunz-Carlisi, Elmar, 50996 Köln (DE); Ochs, Sebastian, 50999 Köln (DE)

(57) **Zusammenfassung**

Die Erfindung betrifft ein Verfahren und eine Anordnung zur individuellen Anleitung mindestens einer Person in Bezug auf physische Bewegungen dieser Person, insbesondere ein Verfahren und eine Anordnung zum interaktiven Fitnesstraining. Das erfindungsgemäße Verfahren umfasst die folgenden Schritte:
- Übermitteln von individuellen Stammdaten und/oder individuellen Bewegungsdaten der Person an mindestens eine Recheneinheit,
- Erzeugen mindestens eines Datensatzes zur individuellen Anleitung der Person zur Ausführung mindestens einer bestimmten Bewegung auf Basis der übermittelten Daten durch die Recheneinheit,
- Übermitteln des Datensatzes an die Person, und
- Anzeigen der Anleitung auf einer Anzeigevorrichtung mittels bewegter Bilder, welche die mindestens eine Bewegung darstellen.

Die erfindungsgemäße Anordnung umfasst mindestens eine Recheneinheit 2, die mindestens eine Kommunikationseinrichtung 5 zum Empfangen von individuellen Stammdaten und/oder individuellen Bewegungsdaten der Person umfasst, und mindestens eine Anzeigevorrichtung, die mit der Recheneinheit 2 zumindest indirekt verbindbar ist.

## Beschreibung

Die Erfindung betrifft ein Verfahren und eine Anordnung zur individuellen Anleitung mindestens einer Person in Bezug auf physische Bewegungen dieser Person, insbesondere ein Verfahren und eine Anordnung zum interaktiven Fitnesstraining.

Die Kosten für das Gesundheitswesen durch Krankheiten, die durch Bewegungsmangel oder falsche Bewegungen verursacht werden, steigen immens. Das Bewegungsverhalten der Bevölkerung muss daher nachhaltig verbessert, die Zunahme von Bewegungsmangel bei Kindern gestoppt und die weitere Verbreitung von Bewegungsmangel verringert werden. Dies kann nur gelingen, wenn das Bewegungsverhalten jedes Einzelnen nachhaltig verbessert und mehr Bewegung dauerhaft in den Alltag integriert wird. Insbesondere Kinder, aber auch viele Erwachsene, bewegen sich zu wenig und sitzen statistisch gesehen für Stunden am Tag vor Computer, Fernseher oder Spielekonsole. Die Kosten, die durch daraus resultierende Krankheiten entstehen, werden in Deutschland zur Zeit mit ca. 30 % aller Gesundheitskosten kalkuliert und betragen damit jährlich mehr als 70 Milliarden Euro. Sport und Bewegung müssen daher im Alltag der Menschen stärker verankert werden, wobei eine professionelle Anleitung zur Ausführung der Bewegungen wünschenswert ist. Wichtig sind dabei vor allem individuelle und zielgruppenspezifische Angebote.

Aus der DE 10 2007 028 686 A1 sind beispielsweise ein Verfahren und ein System zum Erfassen eines Bewegungsprofils einer Person bekannt, welche die Kontrolle und Auswertung der körperlichen Aktivitäten einer Person ermöglichen. Das System umfasst einen Bewegungssensor, eine Feedbackeinrichtung, eine Datenverarbeitungseinrichtung, eine Speichereinrichtung, einen Komparator und eine Prozessoreinheit. Der Bewegungssensor erfasst dabei die Aktivitätsdaten einer Person und leitet die entsprechenden Daten bzw. Signale an die Datenverarbeitungseinrichtung weiter, die anhand der Aktivitätsdaten ein Bewegungsprofil generiert. Dieses Bewegungsprofil wird mittels des Komparators mit einem in der Speichereinrichtung abgelegten Ziel-Bewegungsprofil verglichen und in einem Vergleichswert erfasst. Die Prozessoreinheit steuert dann die Feedbackeinrichtung in Abhängigkeit von diesem Vergleichswert, so dass die Feedbackeinrichtung die sich aus dem Vergleichswert ergebenden Informationen in Form von wahrnehmbaren Signalen an die Person übermitteln kann. Dieses Verfahren hat aber den Nachteil, dass der Person immer nur eine Auswertung der bereits erfolgten Bewegungen bzw. Aktivitäten präsentiert wird, ohne dass sie eine Anleitung zur Ausführung sinnvoller und korrekter Bewegungen erhält. Insbesondere ermöglichen das bekannte Verfahren und die bekannte Vorrichtung kein interaktives Training der Person, da diese keine konkrete, situationsbezogene Anleitung erhält, sondern die übermittelten Daten selbst auswerten und in sinnvolle Bewegungen umsetzen muss. Die Anzeige der Daten durch die Feedbackeinrichtung erschöpft sich dabei in der Wiedergabe einfacher Graphiken und Diagramme.

Es ist Aufgabe der Erfindung, ein Verfahren und eine Anordnung zur individuellen Anleitung mindestens einer Person in Bezug auf physische Bewegungen dieser Person zu schaffen, welche die genannten Nachteile vermeiden und die eine interaktive, situationsbezogene und anschauliche Anleitung der Person zur Ausführung nützlicher Bewegungen bzw. Bewegungsabläufe ermöglichen.

Die Aufgabe wird erfindungsgemäß durch ein Verfahren gelöst, das die folgenden Schritte umfasst:
Übermitteln von individuellen Stammdaten und/oder individuellen Bewegungsdaten der Person an mindestens eine Recheneinheit,
   Erzeugen mindestens eines Datensatzes zur individuellen Anleitung der Person zur Ausführung mindestens einer bestimmten Bewegung auf Basis der übermittelten Daten durch die Recheneinheit,
   Übermitteln des Datensatzes an die Person, und
   Anzeigen der Anleitung auf einer Anzeigevorrichtung mittels bewegter Bilder, welche die mindestens eine Bewegung darstellen.

Das Übermitteln der für ein effektives Training relevanten Daten an eine Recheneinheit, die Verarbeitung dieser Daten durch die Recheneinheit und das Übermitteln der verarbeiteten Daten an die Person ermöglichen in besonders vorteilhafter Weise eine interaktive und situationsbezogene Anleitung dieser Person zur Ausübung sinnvoller und effektiver Bewegungen bzw. Bewegungsabläufe. Die Person erhält somit ein orts- und zeitunabhängiges, individuelles Training für einfache und in den Alltag integrierbare körperliche Aktivitäten und beispielsweise auch eine zielorientierte, gesunde Ernährung. Das erfindungsgemäße Verfahren vermittelt dem Nutzer, welche Bewegungen und beispielsweise auch Essgewohnheiten sinnvoll für das Erreichen der persönlichen Ziele sind und was langfristig zur Etablierung eines aktiven, gesunden Lebensstils erforderlich ist. In vorteilhafter Weise erfolgt die Anleitung der Person auf einer Anzeigevorrichtung mittels bewegter Bilder, welche die mindestens eine Bewegung darstellen, so dass eine sehr anschauliche Anleitung der Person zur Ausführung nützlicher Bewegungen bzw. Bewegungsabläufe gewährleistet ist. So wird der Person anschaulich vorgeführt, wie die Bewegungen durchgeführt werden sollen, um den höchsten Energieverbrauch zu erzeugen bzw. Fehler bei der Ausführung zu vermeiden. Mittels visueller Auswertung kann die Person zudem ihre persönliche Entwicklung (Fitnessstand, Grundverbraucherhöhung, etc.) einfach und effektiv nachvollziehen.

In besonders vorteilhafter Ausgestaltung des erfindungsgemäßen Verfahrens ist vorgesehen, dass die Anleitung der Person mittels eines virtuellen, graphisch darstellbaren Trainers erfolgt, vorzugsweise eines Avatars. Hierdurch wird die Darstellung der auszuführenden Bewegungen besonders deutlich und anschaulich, so dass die Person zusätzliche Motivation erhält, die Übungen auszuführen und darüber hinaus ein effektives und fehlerfreies Training ermöglicht wird.

Zur weiteren Optimierung der Anleitung kann die Anleitung der Person zusätzlich akustisch erfolgen, so dass bestimmte Empfehlungen beispielsweise auch durch eine Sprachausgabe unterstützt und erläutert werden können.

In weiterer vorteilhafter Ausgestaltung des erfindungsgemäßen Verfahrens ist vorgesehen, dass die individuellen Stammdaten und/oder Bewegungsdaten vor dem Übermitteln an die Recheneinheit mittels einer Datenverarbeitungsvorrichtung aufbereitet werden. Auf diese Weise kann die Auswertung der Daten durch die Recheneinheit effektiver und schneller erfolgen. Darüber hinaus wird dem Nutzer ermöglicht, die Daten zusätzlich zu individualisieren und an persönliche Gegebenheiten und Bedürfnisse anzupassen.

In besonders vorteilhafter Ausgestaltung des erfindungsgemäßen Verfahrens ist vorgesehen, dass das Übermitteln der individuellen Stammdaten und/oder Bewegungsdaten an die Recheneinheit mittels mindestens einer Daten- und/oder Telekommunikationsverbindung erfolgt, vorzugsweise mittels einer Internet-, Bluetooth-, Radiofrequenz, WLAN- und/oder GPRS/UMTS-Verbindung. Auch die Anleitung der Person kann in vorteilhafter Weise mittels mindestens einer Daten- und/oder Telekommunikationsverbindung, vorzugsweise mittels einer Internet-, Bluetooth-, Radiofrequenz, WLAN- und/oder GPRS/UMTS-Verbindung, übermittelt werden. Das erfindungsgemäße Verfahren ist also sehr flexibel und kann orts- und zeitunabhängig ausgeführt werden.

In weiterer vorteilhafter Ausgestaltung der Erfindung kann die Person mit mindestens einer mobilen Bewegungserfassungsvorrichtung zum Erfassen physischer Bewegungen ausgestattet werden und die Bewegungsdaten können mit Hilfe dieser Bewegungserfassungsvorrichtung generiert werden. Auf diese Weise können die individuellen Aktivitäten, wie beispielsweise alltägliche Bewegungen im häuslichen Umfeld, beruflichen Umfeld, in der Freizeit und beim Sport, erfasst und mit den persönlichen Zielen und Vorgaben der Person abgeglichen, bewertet und der Person ubiquitär im Sinne eines Feedback-Systems zur Verfügung gestellt werden. Die Daten des Energieverbrauchs, die über den Grundverbrauch hinausgehen, entstehen bei jedem Bewegungsablauf. Diese Bewegungsabläufe sind nicht primär sportbezogen, sondern umfassen auch Aktivitäten des Alltags und der Freizeit, denen ebenfalls präventive Bedeutung zugemessen wird. Mittels eines mobilen Endgerätes, einem sog, Messwertnehmer (z. B. mobile Bewegungsmesser, Schrittzähler, Aktivitätssensoren, etc.), oder händisch können die ausgeführten Bewegungen ermittelt und in das System eingespeist werden. Ein mobiler Bewegungsmesser kann beispielsweise je nach derzeitiger Lage der Person zum Erdboden und dem Takt des Bewegungsimpulses berechnen, welche Bewegung durch diese Person gerade ausgeübt wird. Eine Datenbank, die jede Art von Bewegung einem bestimmten Energiewert zuordnet, kann nun den Energieverbrauch berechnen, den Stammdaten zuordnen und entsprechend der Zielvorgaben auswerten. Die Bewegungen können dann dem jeweiligen Kontext zugeordnet werden und erfahren hierdurch verschiedene Wertigkeiten.

Erfindungsgemäß bevorzugt wird mindestens ein aus den Bewegungsdaten ermittelter Istwert mit mindestens einem Sollwert verglichen, der aus den individuellen Stammdaten der Person und mindestens einem Vergleichswert aus mindestens einer Datenbank ermittelt wird, wobei mindestens ein Datensatz zur individuellen Anleitung der Person zur Ausführung mindestens einer bestimmten Bewegung auf Basis des Ist-Sollwert-Vergleichs erzeugt wird. Bei dem Ist-Sollwert-Vergleich kann dabei vorzugsweise zumindest ein aus den Bewegungsdaten ermittelter Kalorienverbrauch mit einem vorgegebenen Kalorienverbrauchsziel verglichen werden, wobei jeder Art von Bewegung ein bestimmter Kalorienwert zugeordnet wird.

Erfindungsgemäß kann der Datensatz zur individuellen Anleitung der Person mindestens einen Parameter umfassen, aus dem Bewegungsformen und/oder Übungen abgeleitet werden, die eine Optimierung der physischen Bewegungen der Person ermöglichen.

Vorzugsweise wird die individuelle Anleitung für die Person auf einem Bildschirm, vorzugsweise eines Personalcomputers, Laptops oder eines mobilen Kommunikationsgeräts, angezeigt. Als mobiles Kommunikationsgerät kann dabei in vorteilhafter Weise ein MPEG-Player, ein Netbook, ein eBook, ein Smartphone, ein MDA oder ein GSM- oder UMTS - Telekommunikationsgerät eingesetzt werden, was die Flexibilität und Ortsunabhängigkeit des erfindungsgemäßen Verfahrens weiter erhöht.

Die Aufgabe wird erfindungsgemäß ferner durch eine Anordnung gelöst, welche mindestens eine Recheneinheit, die mindestens eine Kommunikationseinrichtung zum Empfangen von individuellen Stammdaten und/oder individuellen Bewegungsdaten der Person umfasst, und mindestens eine Anzeigevorrichtung, die mit der Recheneinheit zumindest indirekt verbindbar ist, umfasst. Die Kommunikationseinrichtung gewährleistet dabei ein schnelles und problemloses Empfangen der Daten durch die Recheneinheit und hierdurch in besonders vorteilhafter Weise eine interaktive und situationsbezogene Anleitung der Person zur Ausübung sinnvoller und effektiver Bewegungen bzw. Bewegungsabläufe. In vorteilhafter Weise erfolgt die Anleitung der Person auf einer Anzeigevorrichtung, so dass eine sehr anschauliche Anleitung der Person zur Ausführung nützlicher Bewegungen bzw. Bewegungsabläufe gewährleistet ist. Die Recheneinheit ist vorzugsweise eine zentrale Recheneinheit (Rechenzentrum), die von mehreren Personen bzw. Nutzern gleichzeitig genutzt werden kann.

In vorteilhafter Ausgestaltung der Erfindung ist mindestens eine Bewegungserfassungsvorrichtung zum Erfassen physischer Bewegungen und Generieren von daraus abgeleiteten Bewegungsdaten vorgesehen, wobei die Bewegungserfassungsvorrichtung vorzugsweise ein Bewegungsmessgerät, insbesondere ein Beschleunigungsmessgerät, ein Schrittzähler oder Aktivitätssensor, ist.

Die Kommunikationseinrichtung umfasst vorzugsweise mindestens eine Schnittstelle für mindestens eine Daten- und/oder Telekommunikationsverbindung, vorzugsweise für eine Internet-, Bluetooth-, Radiofrequenz, WLAN- und/oder GPRS/UMTS-Verbindung. Die erfindungsgemäße Anordnung ist hierdurch sehr flexibel und kann orts- und zeitunabhängig eingesetzt werden.

Vorzugsweise umfasst die Recheneinheit mindestens eine Datenbank und/oder ist mit mindestens einer Datenbank verbunden oder verbindbar, so dass die individuellen Stammdaten und/oder individuellen Bewegungsdaten mit Daten aus der Datenbank verglichen werden können. Die Recheneinheit kann beispielsweise mindestens ein Bauelement (z. B. Komparator) umfassen, das einen Vergleich mindestens eines aus den Bewegungsdaten ermittelten Istwertes mit mindestens einem Sollwert, der aus den individuellen Stammdaten der Person und mindestens einem Vergleichswert aus mindestens einer Datenbank ermittelt wird, durchführen kann.

Die Anzeigevorrichtung ist vorzugsweise mit der Recheneinheit mittels der Kommunikationseinrichtung verbindbar und kann beispielsweise mindestens ein Bildschirm einer Datenverarbeitungsvorrichtung und/oder eines mobilen Kommunikationsgeräts sein, vorzugsweise eines MPEG-Players, Netbooks, eBooks, Smartphones, MDAs oder eines GSM- oder UMTS - Telekommunikationsgeräts. Die erfindungsgemäße Anordnung ist hierdurch sehr flexibel und ortsunabhängig einsetzbar, wobei in besonders vorteilhafter Ausgestaltung der Erfindung bereits vorhandene Mobilfunkgeräte der zu trainierenden Person verwendet werden können.

Die Erfindung betrifft also insbesondere die Verwendung der erfindungsgemäßen, oben beschriebenen Anordnung zur interaktiven, individuellen Anleitung von Personen in Bezug auf deren physische Bewegungen.

Die Erfindung umfasst auch ein Datenverarbeitungsprogramm zur Steuerung des erfindungsgemäßen Verfahrens sowie ein Datenverarbeitungsprogramm zur Steuerung der erfindungsgemäßen Anordnung und/oder mindestens einer oder mehrerer ihrer Komponenten, vorzugsweise ein Datenverarbeitungsprogramm zur Steuerung der Bewegungserfassungsvorrichtung, der Recheneinheit, der Kommunikationseinrichtung und/oder der Anzeigevorrichtung. Vorzugsweise ist die Software eine webbasierte Anwendung und hat eine modulare Basis. Es werden Stammdaten der Person eingegeben (z. B. Alter, Gewicht, Kaloriengrundverbrauch, Gesundheitsstatus, Krankheitsbilder, etc.), welche als Grundlage für die Individualisierung verwendet werden. Auf Basis der persönlichen Angaben kann beispielsweise ein Kalorienverbrauchsziel definiert werden, das sich aus präventivmedizinischen Langzeituntersuchungen ableitet. Damit erhalten die Nutzer einen Handlungsrahmen, der die Grundlage für den regelmäßigen Abgleich mit den tatsächlich absolvierten Bewegungsaktivitäten bildet. Auf diese Weise wird in vorteilhafter Weise ein interaktiver Ist-Sollwert-Vergleich ermöglicht, der auf Dauer zu einer Etablierung regelmäßiger Aktivitäten und damit zu einer Umstellung hin zu einem aktiven Lebensstil führen soll.

Die Erfindung umfasst auch einen Datenträger, auf dem das erfindungsgemäße Datenverarbeitungsprogramm in computerlesbarer Form gespeichert ist.

Die Erfindung wird im Weiteren anhand nachfolgenden Beispiele und der folgenden Abbildungen beispielhaft näher erläutert.

Es zeigen
- Figur 1: eine schematische Darstellung einer ersten beispielhaften Ausfüh- rungsform einer erfindungsgemäßen Anordnung,
- Figur 2: eine schematische Darstellung einer zweiten beispielhaften Ausfüh- rungsform einer erfindungsgemäßen Anordnung,
- Figur 3: eine schematische Darstellung einer dritten beispielhaften Ausfüh- rungsform einer erfindungsgemäßen Anordnung,
- Figur 4: verschiedene beispielhafte Abbildungen eines Avatars zur Anleitung einer Person zur Ausführung verschiedener Bewegungen und
- Figur 5: eine beispielhafte Abbildung einer Bildschirmoberfläche zur Auswahl verschiedener zu trainierender Muskelgruppen.

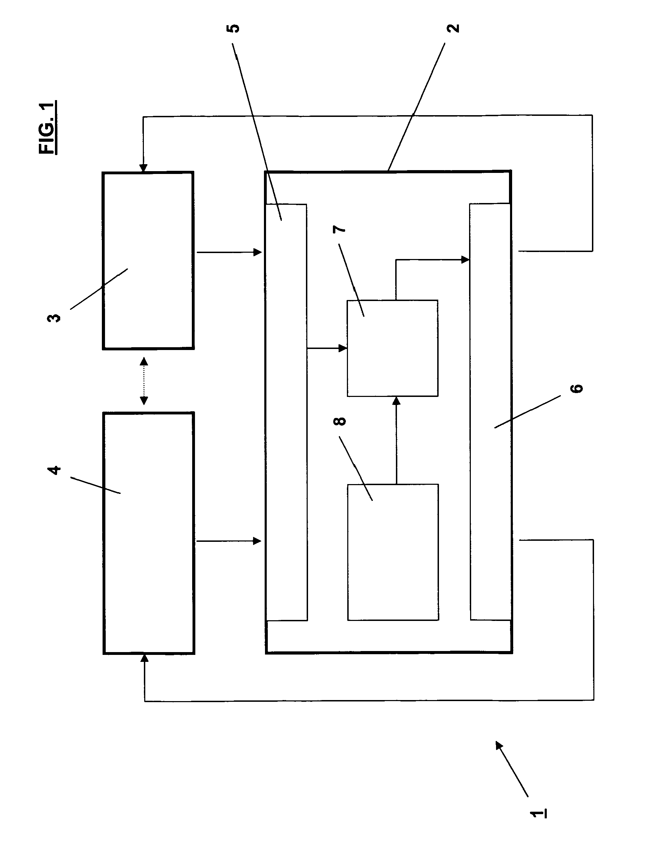

**Figur 1** zeigt eine schematische Darstellung einer besonders vorteilhaften Ausführungsform einer erfindungsgemäßen Anordnung 1. Die Anordnung 1 umfasst eine Recheneinheit 2, die mit einem Kommunikationsgerät 3 und einer Datenverarbeitungsvorrichtung 4 verbunden oder zumindest verbindbar ist. Die Recheneinheit 2 umfasst mindestens eine erste Kommunikationseinrichtung 5 (Input), über die Daten bzw. Signale vom Kommunikationsgerät 3 (z. B. MPEG-Player, ein Netbook, ein eBook, ein Smartphone, ein MDA oder ein GSM- oder UMTS - Telekommunikationsgerät) und/oder der Datenverarbeitungsvorrichtung 4 (z. B. Personalcomputer, Laptop, etc.) in die Recheneinheit 2 eingespeist werden können, und mindestens eine zweite Kommunikationseinrichtung 6 (Output), über die Daten bzw. Signale von der Recheneinheit 2 an das Kommunikationsgerät 3 und/oder die Datenverarbeitungsvorrichtung 4 übermittelt werden können. Erfindungsgemäß kann eine zu trainierende Person individuelle Stammdaten und Bewegungsdaten manuell in die Datenverarbeitungseinrichtung 4 und/oder das Kommunikationsgerät 3 eingeben und über eine geeignete Daten- und/oder Telekommunikationsverbindung an die Recheneinheit 2 übermitteln, wo sie beispielsweise in einer Speichereinrichtung abgelegt werden können. Die Daten- und/oder Telekommunikationsverbindung ist vorzugsweise eine Internet-, Bluetooth-, Radiofrequenz, WLAN- und/oder GPRS/UMTS-Verbindung. Die individuellen Stammdaten und/oder die Bewegungsdaten können dabei vor dem Übermitteln an die Recheneinheit 2 beispielsweise noch mittels der Datenverarbeitungsvorrichtung 4 aufbereitet werden, um eine schnellere und interaktive Verarbeitung der Daten in der Recheneinheit 2 zu ermöglichen. Alternativ können die Bewegungsdaten auch durch eine Bewegungserfassungsvorrichtung (z. B. Beschleunigungssensor, Schrittzähler (Pedometer), Aktivitätssensor oder Ähnliches) automatisch generiert werden, wobei diese Bewegungserfassungsvorrichtung beispielsweise in das Kommunikationsgerät 3 integriert oder an dieses zumindest anschließbar sein kann. Grundsätzlich kann die erfindungsgemäße Anordnung 1 aber auch ohne Bewegungserfassungsvorrichtung und ohne Kommunikationsgerät 3 betrieben werden, wobei die Eingabe der Bewegungsdaten und die Anzeige der Bewegungsanleitungen dann ausschließlich mittels der Datenverarbeitungsvorrichtung 4 erfolgt.

Die Recheneinheit 2 umfasst ferner eine Komparatoreinheit 7, mittels der mindestens ein aus den in die Recheneinheit 2 eingespeisten Bewegungsdaten ermittelter Istwert mit mindestens einem Sollwert verglichen wird. Der Sollwert wird aus den in die Recheneinheit 2 eingespeisten individuellen Stammdaten der Person und mindestens einem Vergleichswert ermittelt, wobei der Vergleichswert aus der Datenbank 8 in die Komparatoreinheit 7 eingespeist wird. Auf diese Weise wird in der Komparatoreinheit 7 mindestens ein Datensatz zur individuellen Anleitung der Person auf Basis des Ist-Sollwert-Vergleichs erzeugt. Dieser Datensatz wird über die Daten- und/oder Telekommunikationsverbindung, vorzugsweise über einer Internet-, Bluetooth-, Radiofrequenz, WLAN- und/oder GPRS/UMTS-Verbindung, an das Kommunikationsgerät 3 und/oder die Datenverarbeitungsvorrichtung 4 übermittelt. Erfindungsgemäß erfolgt die Anleitung der Person bzw. des Nutzers dann durch die Wiedergabe bewegter Bilder auf einer Anzeigevorrichtung, vorzugsweise einem Monitor bzw. Bildschirm des Kommunikationsgeräts 3 und/oder der Datenverarbeitungsvorrichtung 4.

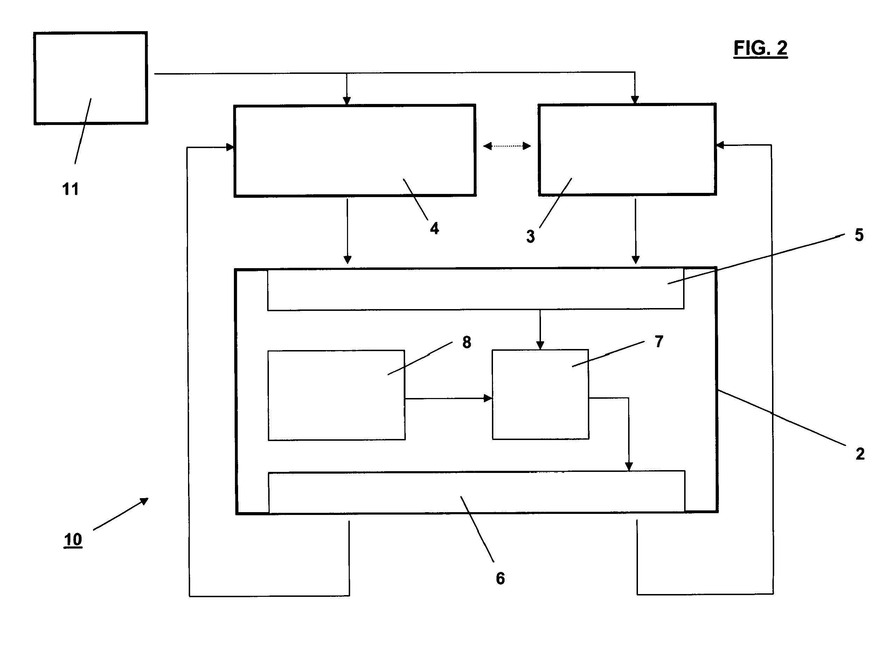

**Figur 2** zeigt eine schematische Darstellung einer weiteren vorteilhaften Ausführungsform einer erfindungsgemäßen Anordnung 10, welche sich von der Anordnung 1 gemäß Figur 1 lediglich durch die separate Bewegungserfassungsvorrichtung 11 unterscheidet (gleiche Komponenten sind hier mit den selben Bezugsziffern wie in Figur 1 versehen). Die Bewegungserfassungsvorrichtung 11 ist mit dem Kommunikationsgerät 3 und/oder der Datenverarbeitungsvorrichtung 4 verbunden oder zumindest verbindbar. Die Bewegungserfassungsvorrichtung 11, bei der es sich beispielsweise um einen Beschleunigungssensor, einen Schrittzähler (Pedometer), einen Aktivitätssensor oder Ähnliches handeln kann, generiert automatisch Bewegungsdaten, die dann an das Kommunikationsgerät 3 und/oder die Datenverarbeitungsvorrichtung 4 und von dort aus an die Recheneinheit 2 übermittelt werden.

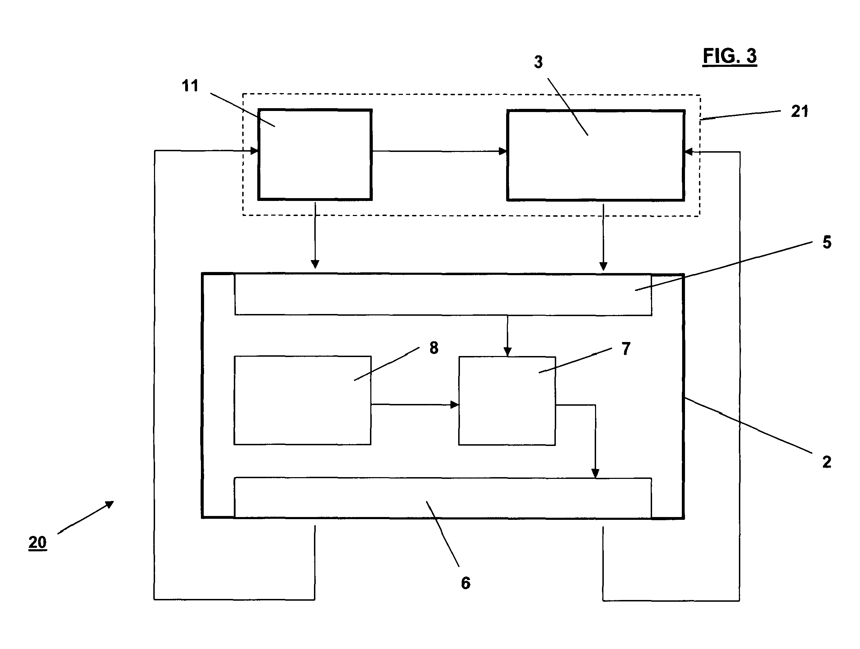

**Figur 3** zeigt eine schematische Darstellung einer weiteren vorteilhaften Ausführungsform einer erfindungsgemäßen Anordnung 20, welche sich von der Anordnung 10 gemäß Figur 2 dadurch unterscheidet, dass hier keine Datenverarbeitungsvorrichtung vorgesehen ist (gleiche Komponenten sind mit den selben Bezugsziffern wie in Figur 2 versehen). Die Bewegungserfassungsvorrichtung 11 ist in dieser Ausführungsform direkt mit dem Kommunikationsgerät 3 und/oder der Recheneinheit 2 verbunden oder zumindest verbindbar. Dabei kann die Bewegungserfassungsvorrichtung 11 gemeinsam mit dem Kommunikationsgerät 3 in einem kompakten Kombinationsgerät 21 kombiniert sein.

### Beispiel 1:

Eine Person telefoniert im Sitzen, was einen minimalen Energieverbrauch impliziert. Steht diese Person aber beim Telefonieren, so ist der Energieverbrauch höher.

Spricht kein Krankheitsbild dagegen, wird der Person folglich durch das erfindungsgemäße System vorgeschlagen, zu stehen oder gar beim Telefonieren hin und herzugehen, sofern ein mobiles Gerät zur Verfügung steht. Abgesehen von dem Kalorienmehrverbrauch fördert dies auch die "Sitz-Steh-Dynamik", also die systematische Unterbrechung monotoner Belastungssituationen, wie sie von ergonomischer Seite gefordert wird.

Für jede mögliche Bewegungsform ist eine Vielzahl von Werten in einer Datenbank hinterlegt, die sich auf die zu trainierende Person (Nutzer), die Situation, den gesundheits-schonensten Bewegungsablauf, die persönlich gesetzten Zielen (z.B. Gewichtsreduktion, Muskelaufbau, Rückenstärkung, etc.) sowie den höchsten Energieaufwand beziehen. Diese Parameter können derart abgestimmt werden, dass der Nutzer die für sie effektivesten Bewegungsformen/Übungen unter Anleitung ausüben kann und darauf angepasste Empfehlungen für eine gesunde Ernährung erhält.

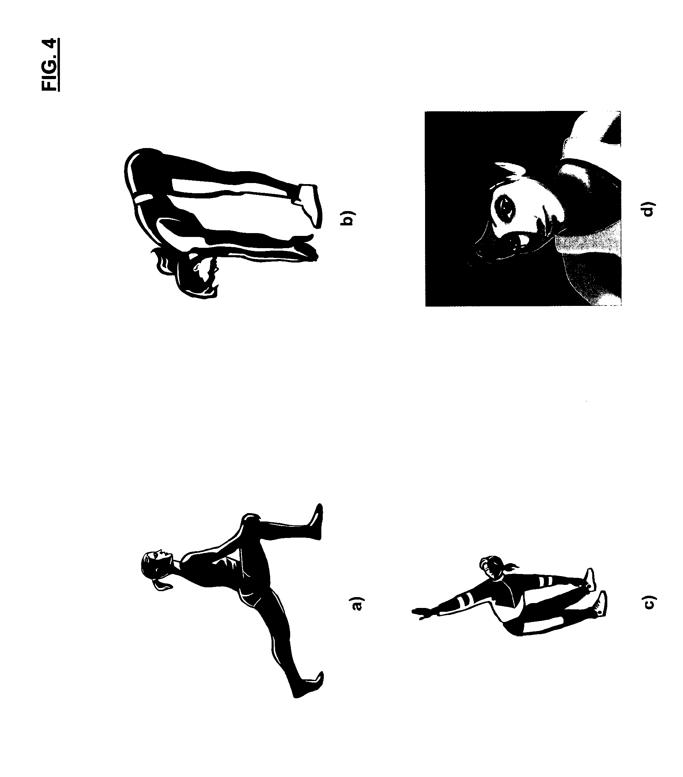

Die Empfehlungen werden durch einen sogenannten "interaktiven Fitnesscoach" in Form von bewegten Bildern dargestellt (**Figur 4 a-c**). Dieser Fitnesscoach kann beispielsweise ein Avatar sein (**Figur 4** **d**), der über ein mobiles Kommunikationsgerät (z. B. Smartphone) in ständigem Kontakt zum Nutzer steht. Der Avatar kann je nach individuellem Wunsch der Person konfiguriert werden, hat eine Sprachausgabe und ist bewegungsanimiert. Der interaktive Fitnesscoach "sagt" dem Nutzer, welche Bewegung und welche Kalorienzufuhr (Ernährung) für ihn in seiner Situation und mit seinen Stammdaten am Besten geeignet ist. Er kann ihm zeigen, wie die Bewegungen ausgeführt werden sollen, um den höchsten Energieverbrauch zu erzielen bzw. Fehler bei der Ausführung zu vermeiden. Mittels visueller Auswertung kann der jeweilige Nutzer des Systems seine persönliche Entwicklung (Fitnessstand, Grundverbraucherhöhung, etc.) nachvollziehen.

Die durch die alltäglichen Bewegungen ermittelten Energieverbrauchswerte können durch zusätzliche Aktivitäten, d. h. kurze Bewegungseinheiten (Workout, Stretching, etc.) "aufgestockt" werden. Auch diese werden durch den Avatar beispielhaft vorgeführt. Der Nutzer erhält so ein orts- und zeitunabhängiges, individuelles Training für einfache und integrierbare körperliche Aktivitäten sowie eine zielorientierte, gesunde Ernährung. Der Fitness- und Ernährungscoach "zeigt" dem Nutzer dabei, welche Bewegungen und Essgewohnheiten sinnvoll für das Erreichen der persönlichen Ziele sind und was langfristig zur Etablierung eines aktiven, gesunden Lebensstils führen kann.

Basierend auf einer explorativen Datenanalyse, d. h. einer systematischen Anwendung von Methoden, die statistisch-mathematisch begründet sind, sowie einem Datenbestand mit sportwissenschaftlichem und sportmedizinischem Hintergrund werden persönliche Empfehlungen und Anleitungen generiert. Die individuellen Aktivitäten, d. h. alltägliche Bewegungen im häuslichen Umfeld, beruflichen Umfeld, Freizeit, und Sport, werden erfasst und mit den persönlichen Zielen und Vorgaben des Nutzers abgeglichen, bewertet und ubiquitär auf einer Anzeigevorrichtung im Sinne eines Feedback-Systems zur Verfügung gestellt.

Das Ausgabemedium kann ein Avatar sein, der sprachgesteuert und sprachausgebend diese Empfehlungen in Kombination mit motivierenden Elementen dem Nutzer kommuniziert. Der Datenbestand ist Bestandteil des webbasierten Systems, welches über eine gesicherte Kommunikationsplattform die Anzeigevorrichtung (z. B. Monitor eines Mobiltelefons) bestückt. Der Dateninput erfolgt über eine Rechnerschnittstelle oder mobil zu den Stammdaten. Der Dateninput zu den Bewegungsabläufen wird über die Bewegungserfassungsvorrichtung (z. B. mobile Bewegungsmesser, Schrittzähler, Aktivitätssensoren, etc) aufgenommen. Die Daten werden über eine drahtlose Schnittstelle (z. B. Bluetooth, RFID, GPRS/UMTS, WLAN, etc.) übermittelt.

Mit Hilfe der erfindungsgemäßen Lösung können also das Thema Prävention nachhaltig umgesetzt und hierfür tagtägliche Bewegungen mittels einer Softwarelösung erfassbar und bewertbar gemacht werden. Ein gesunder Lebensstil soll hiermit gefördert und es soll gezeigt werden, wie einfach es ist, Alltagsbewegungen zu nutzen, zu verstärken und messbar zu machen. Die Bewegungen werden registriert, in bewertbare Energieeinheiten umgewandelt und das System ordnet der Person Handlungs- und Ernährungsempfehlungen zu, welch dass anschaulich dargestellt werden.

### Beispiel 2:

Das erfindungsgemäße Datenverarbeitungsprogramm ist eine neue, einfach zu bedienende Software-Applikation, die zu regelmäßiger Bewegung motiviert und anleitet. In einer bevorzugten Ausführungsform wendet sich die Anwendung speziell an Personen mit Büro-Arbeitsplätzen, also "Sitz-Menschen". Im Mittelpunkt des Produkts steht der Gesundheitsfaktor Bewegung. Dem Nutzer ("User") wird klar, dass jede einzelne Bewegung ein Schritt auf dem Weg zu besserer Gesundheit und Fitness darstellt. Vom Programm angeleitet, erreicht er über den Tagesablauf in kleineren "Bewegungshäppchen" einen Energie-Mehrverbrauch, der in der Summe präventivmedizinisch wertvoll ist. Die Aktivitäten können quasi nebenbei in den Tagesablauf integriert werden, ohne dabei die Arbeitszeit signifikant (ca. 5-10 Minuten) zu belasten. Das Kernziel besteht darin, einen aktiveren (= bewegteren) Lebensstil über die Arbeitswelt zu initiieren und diesen mittel- und langfristig zu etablieren.

Die Erfindung zielt dabei auf die beiden Hauptprobleme ab, die in der modernen Arbeitswelt durch fehlende Bewegung bzw. einseitige Belastungen beim Sitzen hervorgerufen werden:
1) Herz-Kreislauf- und Stoffwechselerkrankungen und
2) vorzeitiger Verschleiß am Stütz- und Bewegungsapparat, insbesondere im Bereich des Rückens.

Durch geeignete Aktivitäten, die sich vom Weg zur Arbeit über die Arbeitszeit bis hin zum Rückweg nach Hause erstrecken, wird diesen Problemen wirkungsvoll entgegen gewirkt. Das Programm bilanziert hierzu die relevanten Aktivitäten jeweils entsprechend ihrer spezifischen Wirksamkeit. So informiert beispielsweise eine allmählich höher lodernde Flamme über den aktuellen Energie-Mehrverbrauch. Die Ausfärbung der Wirbelsäule dabei zeigt an, ob und in wieweit der Rücken genügend Bewegung erfahren hat. Die Anzeige/Bewertung erfolgt auf einen Blick über die zentrale Mittelsäule nach dem "Ampelprinzip". Diese bewusst einfache und plakative Darstellung liefert ein individualisiertes Feedback, indem die persönlichen Angaben und Ziele im Hintergrund mit einer differenzierten, auf ergospirometrischen Messungen basierenden Datenbank stetig abgeglichen werden.

Über den Startbildschirm lassen sich alle Programmfunktionen koordinieren. Die Aktivitäten werden über die entsprechenden "Buttons" aufgerufen, in Minuten-Schritten dokumentiert, ausgewertet und angezeigt (siehe Tabelle 1).

**Tabelle 1**

| **Menü-Button** | **Kurzbeschreibung (mit Fallbeispiel 80 kg)** | **(kcal)** |
|---|---|---|
| Fitness-Start | Aktivitäten auf dem Weg zur Arbeit und zurück; z.B. 2x5 Min. strammes Gehen | 79 |
| Mobil-Faktor | Kleine Manöver für mobiles Arbeiten; z.B. 30 Min. bewegt-stehend telefonieren | 31 |
| Step-Training | Treppen aktivieren Muskeln und Herz-Kreislaufsystem, z.B. 2 x 5 Etagen | 32 |
| Bonus-Meilen | Kleine Erledigungen zu Fuß, Spaziergang in der Mittagspause, insges. 18 Min | 82 |
| Mini-Workout | 2 Minuten gezielte Kräftigung bringen einen "Rückenbonus" | 8 |
| Stretching-Pause | 2 Minuten zur Beweglichmachung bringen weitere Rücken-Bonuspunkte | 5 |
| Meeting-Break | Die Funktion für die gemeinsame Aktivpause zur Auflockerung von Meetings | - |
| **Bilanz** | Energie-Mehrverbrauch bei 4 Minuten Extra-Zeitaufwand: Energie-Soll erfüllt | 237 |

Die hier beispielhaft aufgeführten Aktivitäten erfordern kaum zusätzlichen Zeitaufwand und gehen daher auch nur geringfügig zu Lasten der Arbeitszeit. Viele Aktivitäten können zudem im Rahmen von Aktivpausen bzw. außerhalb der Arbeitszeit stattfinden.

Insgesamt macht die Ausführung des erfindungsgemäßen Verfahrens bewusst, wie einfach es im Grunde ist, auch in der modernen Büro-Arbeitswelt auf ein gesundes Bewegungsmaß zu kommen. Das erfindungsgemäße Verfahren soll auf spielerische Weise eine "Initialzündung" hervorrufen und als Motivator und Reminder im Sinne dauerhafter Aktivität dienen.

### Beispiel 3:

Regelmäßige körperliche Aktivitäten sind von zentraler Bedeutung für die motorische Entwicklung von Kindern und Jugendlichen aller Alterklassen. Umgekehrt belegen aktuelle Studien zunehmend gesundheitliche bzw. motorische Defizite, die auf mangelnde Bewegungsreize zurückzuführen sind. So weisen ca. 60% der Kinder und Jugendlichen Haltungsschäden auf, ca. 30-40% zeigen Koordinationsschwächen. In Verbindung mit einer ungesunden Ernährung ergeben sich zudem Stoffwechselerkrankungen: so sind ca. 15 Prozent der 3-17Jährigen übergewichtig, ca. 6 Prozent adipös.

Eine bevorzugte Ausführungsform des erfindungsgemäßen Verfahrens wendet sich an Kinder und Jugendliche im Alter zwischen 8 und 17 Jahren, die zu regelmäßiger Bewegung - definiert gemäß internationaler Empfehlungen - mit Spaß motiviert und angeleitet werden sollen. Zunächst wird das Bewegungsverhalten anhand einer typischen Woche analysiert. Fitnesstests geben weiteren Aufschluss über individuelle Stärken und mögliche Defizite. Mit diesem Wissen ausgestattet, erhalten die Kinder und Jugendlichen - unter Berücksichtigung ggf. vorhandener Defizite im Bereich des Halte- und Bewegungsapparates sowie der kardiopulmonalen Leistungsfähigkeit - individuelle Übungs- und Trainingspläne, mit dem Ziel, diese Aktivitäten auf Dauer zu etablieren. Das erfindungsgemäße Datenverarbeitungsprogramm agiert damit im Sinne eines interaktiven "Personal Trainers", der spielerisch anleitet, aber nicht belehrt. Ein spezieller Anreiz zur regelmäßigen Aktivität und Überprüfung des Trainingsfortschritts besteht in einem Punktesystem, das auch die Grundlage für Bonifikationen darstellt. Zusätzliche Informationen zu den speziellen Vorteilen der gängigen Freizeitsportarten, zur gesunden Ernährung sowie ein Bericht ("Report") (auch und gerade) für die Eltern runden das Programm ab.

Die wesentlichen motorischen Grundeigenschaften können entweder per Selbsttest ermittelt oder unter professionellen Bedingungen auf Events gemessen, als "Fitness-Olympiade" organisiert und online übertragen werden. Alle Ergebnisse werden grafisch ausgewertet, interpretiert und als Verlauf dokumentiert. Dabei finden sowohl eine Einzel- als auch eine Gesamtauswertung statt. Auf Basis der Testergebnisse wird ein individueller Trainingsplan mit den wichtigsten Übungen erstellt. Die Übungen werden mittels bewegter Bilder (Avatare) realistisch dargestellt (**Figur 4**).

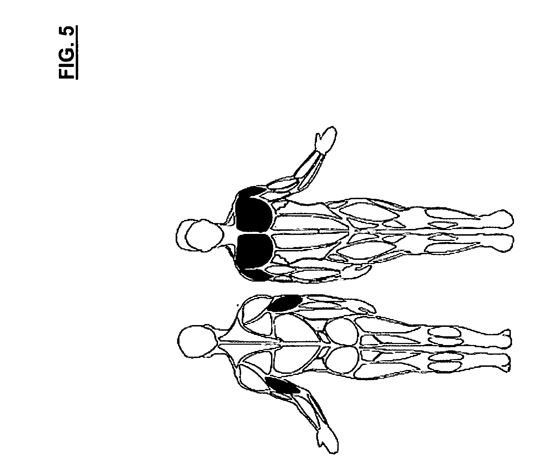

Ein "Menü" des erfindungsgemäßen Datenverarbeitungsprogramms widmet sich der Trainingspraxis. Hier sind alle Übungen aufrufbar, im Detail zu betrachten (Avatar-Darstellung) und als Übungsprogramm kombinierbar. Auch zielgruppenspezifische Trainingsprogramme sind hier hinterlegt und können abgerufen werden. Je Alterklasse (Kids oder Teens) und Geschlecht werden anhand der Stammdaten "Avatar-Charactere" zugeordnet (**Figur 4** **d**). Der entsprechende "Personal-Trainer" führt die Übungen in bewegten Bildern und unterschiedlichen Darstellungen/Perspektiven vor (**Figuren 4 a-c**). Grundlage der Trainingsprogramme bildet eine Übungsdatenbank, die in die Bereiche Kräftigung, Beweglichmachung und Koordinationstraining unterteilt ist. In dieser Untergliederung lassen sich die Übungen in unmittelbarem Bezug zum Fitness-Ergebnis-Check zuordnen. Die Übungen in den einzelnen Rubriken können über einen "Muskelmann" (Front- und Rückansicht) markiert und abgerufen sein, damit diese vom Nutzer ("User") intuitiv (ohne anatomisches Vorwissen) aufgerufen werden können (**Figur 5**). Die Auswahl der Muskelareale ist so angelegt, dass ein Training der wesentlichen, großen Muskelgruppen abgedeckt und ein harmonisches Ganzkörpertraining gewährleistet ist. Je nach aufgerufener Muskelpartie werden die verfügbaren Übungen angezeigt. Darüber hinaus lassen sich Zusatzinformationen zur Bezeichnung und Funktion der jeweiligen Muskeln abrufen ("Muskel-Guide").

Aufgerufene Übungen werden vom "Personal-Trainer" in Echtzeit (mit Vor- und Rücklauf sowie Standbild) vorgeführt (**Figur 4**). Mit unterschiedlichen Kameraperspektiven wird eine zusätzliche Verdeutlichung des Bewegungsablaufs erreicht. Kurze Textbeschreibungen mit Informationen zu den wichtigsten Kriterien einer korrekten Bewegungsausführung unterstützen den Lernprozess. Um ein individuelle Zuordnung/Empfehlung geeigneter Übungen zu erreichen, werden die Übungen in der Datenbank mit Zusatz-Informationen belegt, die sich aus den Stammdaten sowie den Ergebnissen des Fitness-Tests zusammensetzen. Aus der Verknüpfung der Informationen werden die individuell geeigneten Übungen herausgefiltert und zu Übungsprogrammen zusammengefasst. Diese können themenbezogen (z.B. Kräftigung) als auch übergreifend (Kraft, Beweglichkeit, Koordination) in unterschiedlicher Anzahl kombiniert werden. Alle Trainingsprogramme stehen als Ausdrucke zur Verfügung.

Dem Nutzer stehen folgende Programmtypen zur Verfügung:
■ Individuelle Trainingspläne (individuell erstellt)
■ Themenbezogene Trainingspläne (konfiguriert)
■ Sportartspezifische Trainingspläne (konfiguriert)

### Individuelle Trainingspläne:

Diese werden anhand der Stammdaten (Alter, BMI = "body mass index") sowie der Resultate des Fitnesstests individuell zusammengestellt. Mit diesem Programm soll eine harmonische Förderung der persönlichen Fitness erreicht werden. Anhand eines in der Datenbank hinterlegten Algorithmus werden die jeweils am besten passenden Übungen ausgewählt und als Programm zusammengestellt.

Der Nutzer erhält auch eine Zusammenfassung der wichtigsten Ergebnisse, Ziele und Empfehlungen. Um den Verlauf des Körpergewichts präzise in den einzelnen Entwicklungsabschnitten verfolgen zu können, findet eine aktuelle Bewertung des BMI in Perzentilen statt.

### Bezugszeichenliste:

- 1: Anordnung
- 2: Recheneinheit
- 3: Kommunikationsgerät
- 4: Datenverarbeitungsvorrichtung
- 5: Kommunikationseinrichtung
- 6: Kommunikationseinrichtung
- 7: Komparatoreinheit
- 8: Datenbank
- 9: -
- 10: Anordnung
- 11: Bewegungserfassungsvorrichtung
- 20: Anordnung
- 21: Kombinationsgerät

## Patentansprüche

1. Verfahren zur individuellen Anleitung mindestens einer Person in Bezug auf physische Bewegungen dieser Person, welches die folgenden Schritte umfasst:
- Übermitteln von individuellen Stammdaten und/oder individuellen Bewegungsdaten der Person an mindestens eine Recheneinheit (2),
- Erzeugen mindestens eines Datensatzes zur individuellen Anleitung der Person zur Ausführung mindestens einer bestimmten Bewegung auf Ba- sis der übermittelten Daten durch die Recheneinheit (2),
- Übermitteln des Datensatzes an die Person, und
- Anzeigen der Anleitung auf einer Anzeigevorrichtung mittels bewegter Bilder, welche die mindestens eine Bewegung darstellen.

2. Verfahren nach Anspruch 1,
**dadurch gekennzeichnet, dass**
die Anleitung der Person mittels eines virtuellen, graphisch darstellbaren Trainers erfolgt, vorzugsweise eines Avatars.

3. Verfahren nach Anspruch 1 oder 2,
**dadurch gekennzeichnet,dass**
die Anleitung der Person zusätzlich akustisch erfolgt.

4. Verfahren nach Anspruch 1, 2 oder 3,
**dadurch gekennzeichnet, dass**
die individuellen Stammdaten und/oder Bewegungsdaten vor dem Übermitteln an die Recheneinheit (2) mittels einer Datenverarbeitungsvorrichtung (4) aufbereitet werden.

5. Verfahren nach einem der vorhergehenden Ansprüche,
**dadurch gekennzeichnet, dass**
das Übermitteln der individuellen Stammdaten und/oder Bewegungsdaten an die Recheneinheit (2) mittels mindestens einer Daten- und/oder Telekommunikationsverbindung erfolgt, vorzugsweise mittels einer Internet-, Bluetooth-, Radiofrequenz, WLAN- und/oder GPRS/UMTS-Verbindung.

6. Verfahren nach einem der vorhergehenden Ansprüche,
**dadurch gekennzeichnet, dass**
die Anleitung der Person mittels mindestens einer Daten- und/oder Telekommunikationsverbindung, vorzugsweise mittels einer Internet-, Bluetooth-, Radiofrequenz, WLAN- und/oder GPRS/UMTS-Verbindung, übermittelt wird.

7. Verfahren nach einem der vorhergehenden Ansprüche,
**dadurch gekennzeichnet, dass**
die Person mit mindestens einer mobilen Bewegungserfassungsvorrichtung (11) zum Erfassen physischer Bewegungen ausgestattet wird und die Bewegungsdaten mit Hilfe dieser Bewegungserfassungsvorrichtung (11) generiert werden.

8. Verfahren nach einem der vorhergehenden Ansprüche,
**dadurch gekennzeichnet, dass**
mindestens ein aus den Bewegungsdaten ermittelter Istwert mit mindestens einem Sollwert verglichen wird, der aus den individuellen Stammdaten der Person und mindestens einem Vergleichswert aus mindestens einer Datenbank (8) ermittelt wird, und dass mindestens ein Datensatz zur individuellen Anleitung der Person zur Ausführung mindestens einer bestimmten Bewegung auf Basis des Ist-Sollwert-Vergleichs erzeugt wird.

9. Verfahren nach Anspruch 8,
**dadurch gekennzeichnet, dass**
bei dem Ist-Sollwert-Vergleich zumindest ein aus den Bewegungsdaten ermittelter Kalorienverbrauch mit einem vorgegebenen Kalorienverbrauchsziel verglichen wird, wobei jeder Art von Bewegung ein bestimmter Kalorienwert zugeordnet wird.

10. Verfahren nach einem der vorhergehenden Ansprüche,
**dadurch gekennzeichnet, dass**
der Datensatz zur individuellen Anleitung der Person mindestens einen Parameter umfasst, aus dem Bewegungsformen und/oder Übungen abgeleitet werden, die eine Optimierung der physischen Bewegungen der Person ermöglichen.

11. Verfahren nach einem der vorhergehenden Ansprüche,
**dadurch gekennzeichnet, dass**
die individuelle Anleitung für die Person auf einem Bildschirm, vorzugsweise eines Personalcomputers, Laptops oder eines mobilen Kommunikationsgeräts (3), angezeigt werden.

12. Verfahren nach Anspruch 11,
**dadurch gekennzeichnet, dass**
das mobile Kommunikationsgerät (3) ein MPEG-Player, ein Netbook, ein eBook, ein Smartphone, ein MDA oder ein GSM- oder UMTS - Telekommunikationsgerät ist.

13. Anordnung (1, 10, 20) zur individuellen Anleitung mindestens einer Person in Bezug auf physische Bewegungen dieser Person,
**gekennzeichnet durch**
mindestens eine Recheneinheit (2), die mindestens eine Kommunikationseinrichtung (5, 6) zum Empfangen von individuellen Stammdaten und/oder individuellen Bewegungsdaten der Person umfasst, und
mindestens eine Anzeigevorrichtung, die mit der Recheneinheit (2) zumindest indirekt verbindbar ist.

14. Anordnung nach Anspruch 13,
**dadurch gekennzeichnet, dass**
mindestens eine Bewegungserfassungsvorrichtung (11) zum Erfassen physischer Bewegungen und Generieren von daraus abgeleiteten Bewegungsdaten vorgesehen ist, wobei die Bewegungserfassungsvorrichtung (11) vorzugsweise ein Bewegungsmessgerät, insbesondere ein Beschleunigungsmessgerät, ein Schrittzähler oder Aktivitätssensor, ist.

15. Anordnung nach Anspruch 13 oder 14,
**dadurch gekennzeichnet, dass**
die Kommunikationseinrichtung (5, 6) mindestens eine Schnittstelle für mindestens eine Daten- und/oder Telekommunikationsverbindung umfasst, vorzugsweise für eine Internet-, Bluetooth-, Radiofrequenz, WLAN- und/oder GPRS/UMTS-Verbindung.

16. Anordnung nach einem der vorhergehenden Ansprüche,
**dadurch gekennzeichnet, dass**
die Recheneinheit (2) mindestens eine Datenbank (8) umfasst und/oder mit mindestens einer Datenbank (8) verbunden oder verbindbar ist.

17. Anordnung nach einem der vorhergehenden Ansprüche,
**dadurch gekennzeichnet, dass**
die Recheneinheit (2) mindestens ein Bauelement umfasst, das einen Vergleich mindestens eines aus den Bewegungsdaten ermittelten Istwertes mit mindestens einem Sollwert, der aus den individuellen Stammdaten der Person und mindestens einem Vergleichswert aus mindestens einer Datenbank ermittelt wird, durchführt.

18. Anordnung nach einem der vorhergehenden Ansprüche,
**dadurch gekennzeichnet, dass**
die Anzeigevorrichtung mit der Recheneinheit (2) mittels der Kommunikationseinrichtung (5, 6) verbindbar ist.

19. Anordnung nach einem der vorhergehenden Ansprüche,
**dadurch gekennzeichnet, dass**
die Anzeigevorrichtung mindestens ein Bildschirm einer Datenverarbeitungsvorrichtung (4) und/oder eines mobilen Kommunikationsgeräts (3) ist, vorzugsweise eines MPEG-Players, Netbooks, eBooks, Smartphones, MDAs oder eines GSM- oder UMTS - Telekommunikationsgeräts.

20. Verwendung der Anordnung (1, 10, 20) nach einem der Ansprüche 13 bis 19 zur interaktiven, individuellen Anleitung von Personen in Bezug auf deren physische Bewegungen.

21. Datenverarbeitungsprogramm zur Steuerung des Verfahrens nach einem der Ansprüche 1 bis 12.

22. Datenverarbeitungsprogramm zur Steuerung der Anordnung (1, 10, 20) nach einem der Ansprüche 13 bis 19 und/oder mindestens einer Komponente der Anordnung (1, 10, 20) nach einem der Ansprüche 13 bis 19, vorzugsweise der Bewegungserfassungsvorrichtung (11), der Recheneinheit (2), der Kommunikationseinrichtung (5, 6) und/oder der Anzeigevorrichtung.

23. Datenträger, auf dem das Datenverarbeitungsprogramm nach Anspruch 21 und/oder 22 in computerlesbarer Form gespeichert ist.
